# EUROPEAN PATENT APPLICATION

(11) **EP 4 410 243 A1**
(43) Date of publication of application: **07.08.2024**
(21) Application number: 22930629.5
(22) Date of filing: 01.12.2022
(51) Int. Cl.: A61F 2/24

(54) **ARTIFICIAL HEART VALVE**

(30) Priority: 11.03.2022 CN 202210238277
(71) Applicant: Shanghai Trulive Medtech Co., Ltd., Shanghai 201206 (CN); Jiangsu Trulive Medtech Co., Ltd., Nantong, Jiangsu 226400 (CN)
(72) Inventor: HAN, Tianyu, Shanghai 201206 (CN); LIU, Xiang, Shanghai 201206 (CN); WEI, Yongqiang, Shanghai 201206 (CN); LI, Lei, Shanghai 201206 (CN)
(74) Representative: Nordmeyer, Philipp Werner
(86) International application number: PCT/CN2022/135854
(87) International publication number: WO 2023/169006

(57) **Abstract**

An artificial heart valve (100), comprising a body stent and an artificial valve leaflet (130), wherein an outflow end of the body stent is connected to an inflow end of the artificial valve leaflet (130), and when the artificial heart valve (100) is implanted in the heart, the body stent is secured to a mitral valve annulus, and the artificial valve leaflet (130) is in sealing contact with an inner side of a native posterior leaflet (21) of a mitral valve leaflet and mates with a native anterior leaflet (22) of the mitral valve leaflet, so as to ensure unidirectional flow of blood in a left atrium (10) and a left ventricle (20). The artificial valve leaflet (130) is used for space occupation and mating, thereby effectively utilizing the healthy native anterior leaflet (22), and improving the mating performance and service life of the valve leaflet.

## Description

### TECHNICAL FIELD

The present invention relates to the technical field of medical devices and, in particular, to an artificial heart valve.

### BACKGROUND

The heart contains four cardiac chambers, wherein the left atrium and left ventricle are on the left side of the heart, and the right atrium and right ventricle are on the right side of the heart. The ventricular inflow tract is formed between the atrium and the ventricle, the left ventricle and the aorta form the left ventricular outflow tract, the right ventricle and the pulmonary artery form the right ventricular outflow tract, and throughout the cardiac cycle, the pumping function of the left atrium and right atrium of the heart usually happens synchronously. Everyone has four valves in their heart, namely the aortic valve connecting the left ventricle and aorta, the pulmonary valve connecting the right ventricle and pulmonary artery, the mitral valve connecting the left atrium and left ventricle, and the tricuspid valve connecting the right atrium and right ventricle. They all function as one-way valves, allowing blood to flow only from one direction to another but not backwards. At the onset of ventricular filling (i.e., diastole), the aortic and pulmonic valves close to prevent backflow from the arteries into the ventricles; shortly thereafter, the mitral and tricuspid valves open to allow unimpeded flow from the atria into the corresponding ventricles. Shortly after the onset of ventricular systole (i.e., ventricular emptying), the tricuspid and mitral valves close normally, which form a seal that prevents backflow from the ventricles into the corresponding atriums.

The structure of the human heart is very complex, especially the mitral valve structure, which is more complex than the aortic valve. The annular shape of the mitral valve structure is irregular, and multiple chordae tendineae in the ventricular cavity seriously interfere with the implantation and positioning of the interventional valve. Therefore, in transcatheter mitral valve replacement (TMVR), catheter intervention is applied so to compress the artificial valve into the delivery system outside the body, and then delivers it to the mitral valve annulus in human body along the vascular path or through the apex of the heart, and then the artificial valve is released and fixed at the mitral valve annulus to replace the native valve. Compared with surgery, TMVR does not require extracorporeal circulation auxiliary devices. It has the advantages of less trauma and faster recovery. The patient's hemodynamic indicators can be significantly improved after surgery. Compared with the transapical approach, the interatrial septum approach through the femoral vein is less traumatic and more widely used.

Although mitral valve replacement technology has developed rapidly, there are still some recognized difficulties in the design of artificial valves. For example, due to the complex structure of the mitral valve (saddle shape, large size, etc.), the anchoring of the stent of the artificial valve is difficult, and the performance problems of artificial valve leaflets (such as biological valve leaflets or mechanical valve leaflets) causes limited service life of existing artificial valve leaflets, especially biological valve leaflets used in interventional treatments, such as pig pericardium, the mechanical properties of which will decrease or even fail due to factors such as impact load and friction when it has been opened and closed for a long time. Not only will their lifespan be shorter than that of the original valve leaflets, but in the long term, the alignment performance of the artificial valve leaflets will also be inferior to that of the original valve leaflets.

### SUMMARY OF THE INVENTION

An object of the present invention is to provide an artificial heart valve that can solve the problems of limited service life of existing artificial valve leaflet and reduced mating performance of artificial valve leaflet.

In order to solve the above problems, the present invention provides An artificial heart valve, comprising a body stent and an artificial valve leaflet, wherein an outflow end of the body stent is connected to an inflow end of the artificial valve leaflet, and when the artificial heart valve is implanted in the heart, the body stent is secured to a mitral valve annulus, and the artificial valve leaflet is in sealing contact with an inner side of a native posterior leaflet of a mitral valve leaflet and mates with a native anterior leaflet of the mitral valve leaflet, so as to ensure unidirectional flow of blood in a left atrium and a left ventricle.

Optionally, the body stent comprises a lug section and a main section that are connected axially, an inflow end of the main section is connected to an outflow end of the lug section, and an outflow end of the main section is connected to the inflow end of the artificial valve leaflet, and wherein the lug section is configured to connect with a delivery system before the artificial heart valve is released, and the main section is configured to anchor the artificial heart valve to the mitral valve annulus.

Further, the main section has a cylindrical structure with two open ends, and the cylindrical structure is formed by a plurality of mesh-structured units connected to each other along axial and radial directions, and wherein an outer peripheral surface of the main section is configured to abut against an inner wall of the left atrium located above the mitral valve annulus.

Further, a plurality of barbs are provided on an outer peripheral surface of the main section, and all the barbs are evenly distributed along a circumferential direction of the main section to assist the main section in anchoring the artificial heart valve to the mitral valve annulus.

Further, the lug section is composed of a plurality of lugs, and all the lugs are evenly distributed along the circumferential direction of the main section.

Further, the lug comprises a connecting portion and two ribs, and wherein the connecting portion is configured to connect to the delivery system, inflow ends of the two ribs are connected to the connecting portion, outflow ends of the two ribs are connected to the main section, and the ribs each extend in a curve from the inflow end to the outflow end.

Optionally, the artificial valve leaflet comprises a valve stent, a covering and a clamping component;
an inflow end of the valve stent is connected to an outflow end of the main section, and is overlapped and arranged inside the outflow end of the main section;
the covering is attached to an outer side of an outflow end of the valve stent, so that an outflow end of the valve stent with the covering attached is configured to be in sealing contact with an inner side of the native posterior leaflet of the mitral valve leaflet and mate with the native anterior leaflet of the mitral valve leaflet; and
the clamping component is connected to the outer side of the outflow end of the valve stent, and the covering exposes the clamping component from outside so that the native posterior leaflet is clamped and secured between the clamping component and the outflow end of the valve stent.

Further, the inflow end of the valve stent has an arc-shaped structure formed by a plurality of mesh-structured units connected together, and a radial diameter of the inflow end of the valve stent is same as a radial diameter of the main section.

Further, the outflow end of the valve stent has an arc-shaped structure formed by a plurality of mesh-structured units connected together; or the outflow end of the valve stent has a cylindrical structure with at least one open end, and the cylindrical structure is formed by a plurality of mesh-structured units connected together;
wherein a radial diameter of the outflow end of the valve stent is smaller than a radial diameter of the inflow end of the valve stent.

Further, the mesh-structured units of the inflow end of the valve stent are matched with and arranged inside the mesh-structured units of the outflow end of the body stent, and the mesh-structured units of the inflow end of the valve stent are connected to the mesh-structured units of the outflow end of the body stent at overlapped connecting points.

Further, the mesh-structured units of the inflow end of the valve stent are same in shape and size as the mesh-structured units of the outflow end of the body stent.

Further, the outflow end of the valve stent has the arc-shaped structure, the covering is attached to a side of the outflow end of the valve stent away from an axis of the main section, or the covering covers an outer surface of the outflow end of the valve stent; or
the outflow end of the valve stent has the cylindrical structure, the covering covers an outer surface of the outflow end of the valve stent.

Further, the covering covers an outer surface of the outflow end of the valve stent, and the covering is filled with filler.

Further, the filler comprises biocompatible foam.

Further, the clamping component is formed by bending a metal rod, the clamping component has two ends, one of which is connected to the outer side of the outflow end of the valve stent, and the other of which extends circumferentially away from an axis of the main section and axially away from the inflow end of the valve stent, and then extends circumferentially away from the axis of the main section and axially towards the inflow end of the valve stent.

Compared to the prior art, the present invention offers the following advantages:
The present invention provides an artificial heart valve, comprising a body stent and an artificial valve leaflet, wherein an outflow end of the body stent is connected to an inflow end of the artificial valve leaflet, and when the artificial heart valve is implanted in the heart, the body stent is secured to a mitral valve annulus, and the artificial valve leaflet is in sealing contact with an inner side of a native posterior leaflet of a mitral valve leaflet and mates with a native anterior leaflet of the mitral valve leaflet, so as to ensure unidirectional flow of blood in a left atrium and a left ventricle. According to the present invention, the artificial valve leaflet is used for space occupation and mating, thereby effectively utilizing the healthy native anterior leaflet, and improving the mating performance and service life of the valve leaflet.

Further, the covering is attached to the outer side of the outflow end of the valve stent, so that only the outflow end of the artificial valve leaflet is covered with the coating, while the body stent is not covered with the coating. In this way, the artificial heart valve occupies a small area of the valve orifice and bears little impact, thereby effectively prolonging the service life of the body stent and the artificial heart valve.

In addition, a plurality of barbs are provided on the outer peripheral surface of the main section, so that the main section can be anchored to the mitral valve annulus, and the clamping component allows the artificial heart valve to be more reliably anchored between the left atrium and left ventricle.

### BRIEF DESCRIPTION OF THE DRAWINGS

Fig. 1 is a schematic diagram showing the three-dimensional structure of an artificial heart valve located in a heart according to an embodiment of the present invention;
Fig. 2a is a schematic diagram showing the three-dimensional structure of an artificial heart valve according to an embodiment of the present invention;
Fig. 2b is a schematic diagram showing the three-dimensional structure of a valve stent according to an embodiment of the present application;
Figs. 3a-3c are schematic diagrams of an artificial heart valve during implantation according to an embodiment of the present invention.

List of reference signs:
10, left atrium; 20, left ventricle; 21, native posterior leaflet; 22, native anterior leaflet; 30, delivery catheter;
100, artificial heart valve; 110, lug section; 111, connecting portion; 112, rib; 120, main section; 130, artificial valve leaflet; 131, valve stent; 1311, first part; 1312, second part; 140, clamping component.

### DETAILED DESCRIPTION

The artificial heart valve according to the present invention is described below in further detail. The invention will now be described in more detail with reference to the accompanying drawings, in which preferred embodiments of the invention are shown, it being understood that those skilled in the art may modify the invention described herein while still achieving the advantageous effects of the invention. Therefore, the following description should be understood as being widely known to those skilled in the art and is not intended to limit the present invention.

In the interest of clarity, not all features of an actual embodiment are described. In the following description, well-known functions and constructions are not described in detail since they would obscure the invention with unnecessary details. It is understood that in the development of any actual embodiment, numerous implementation details must be made to achieve the developer's particular goals, such as changing from one embodiment to another in accordance with relevant system or relevant business constraints. Additionally, it should be appreciated that such development efforts may be complex and time consuming, but are simply routine tasks for those skilled in the art.

In order to make the purpose and features of the present invention more obvious and understandable, embodiments of the present invention will be further described below with reference to the accompanying drawings. It is noted that the figures are provided in a very simplified form not necessarily presented to scale, with the only intention of facilitating convenience and clarity in explaining the embodiments. In this description, the term "or" is generally employed in its sense including "and/or" unless the content clearly dictates otherwise. The terms "inner", "outer", and similar expressions used herein are for illustrative purposes only and do not represent the only implementations. In this description, the end close to blood flowing into the left atrium is defined as the inflow end, and the end close to blood flowing out of the left ventricle is defined as the outflow end.

The left heart structure includes the left atrium, left ventricle, the mitral valve annulus at the junction between the left atrium and the left ventricle, the mitral valve leaflets attached to the mitral valve annulus, and the papillary muscles and their chordae tendineae located in the left ventricle. A healthy heart pumps blood from the left atrium into the left ventricle, with mitral valve leaflets ensuring one-way flow, and ultimately out from the outflow tract of left ventricular. The one-way flow of blood in the heart cannot be completely ensured due to mitral valve insufficiency caused from diseased mitral valve leaflets, leading to the occurrence of mitral valve regurgitation. The mitral valve leaflets include the native anterior leaflet and the native posterior leaflet, and the chordae tendineae of the native posterior leaflet is connected on both sides to the native posterior leaflet and its papillary muscles respectively. A gap is formed between two adjacent ones of the chordae tendineae of the native posterior leaflet. The diseased mitral valve leaflet is usually the native posterior leaflet. Therefore, the diseased native posterior leaflet cannot function normally, resulting in insufficiency of the mitral valve.

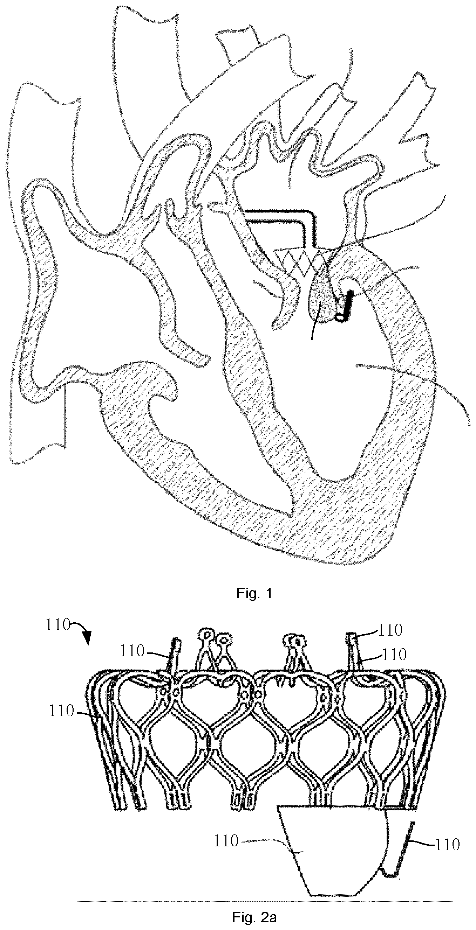

Fig. 1 is a schematic diagram showing the three-dimensional structure of an artificial heart valve located in a heart according to the present embodiment. Fig. 2a is a schematic diagram showing the three-dimensional structure of an artificial heart valve according to the present embodiment. As shown in Figs. 1 and 2a, the present embodiment provides an artificial heart valve 100, which is configured to be implanted into the heart through a catheter and finally anchored between the left atrium 10 and left ventricle 20, in order to replace parts of the mitral valve leaflets to ensure one-way flow of blood and thus to treat mitral valve insufficiency caused by diseased native posterior leaflet 21, that is, to prevent blood from flowing back from the left ventricle 20 into the left atrium 10.

The artificial heart valve 100 includes a body stent and an artificial valve leaflet 130 that are axially connected. Here, the body stent and the artificial valve leaflet 130 are arranged in sequence from the inflow end to the outflow end of the artificial heart valve 100, that is, the outflow end of the body stent is connected to the inflow end of the artificial valve leaflet.

After the artificial heart valve 100 is implanted in the heart, the body stent is secured to the mitral valve annulus, and the artificial valve leaflet 130 is secured to the native posterior leaflet 21 of the mitral valve. Specifically, the artificial valve leaflet 130 is in sealing contact with the inner side of the native posterior leaflet 21 of the mitral valve leaflets, and is used to replace the native posterior leaflet 21 to mate with the native anterior leaflet 22, thereby ensuring one-way flow of blood in the left atrium 10 and the left ventricle 20. It should be noted that the "axial" refers to a direction of the artificial heart valve 100 that is parallel to the blood flow direction after it is implemented into the heart. In this way, the direction perpendicular to the blood flow direction is the radial direction of the artificial heart valve 100.

The body stent includes a lug section 110 and a main section 120 that are axially connected. The inflow end of the main section 120 is connected to the outflow end of the lug section 110, and the outflow end of the main section 120 is connected to the inflow end of the artificial valve leaflet 130 to carry the artificial valve leaflet 130. The main section 120 is configured to anchor the artificial heart valve 100 to the mitral valve annulus. The lug section 110 is configured to connect to the delivery system before the release of the artificial heart valve so that the artificial heart valve 100 can be securely loaded to the distal end of the delivery catheter 30 of the delivery system before release.

The lug section 110 is composed of a plurality of lugs, all of which are evenly distributed along the circumferential direction of the main section 120. Specifically, the inflow ends of all the lugs converge towards the axis of the main section 120, and the lugs each extend from its inflow end to outflow end gradually away from the axis of the main section 120 to connect to the inflow end of the main section 120, so that the circumferential size of the lug is small, and it is easier for the delivery system to retract the body stent, It can also increase the circumferential stiffness of the body stent.

Further, the lug includes a connecting portion 111 and two ribs 112 along the axial direction. The inflow ends of the two ribs 112 are connected together and further connected to the connecting portion 111, and the outflow ends of the two ribs 112 are connected to the main section 120. The rib 112 extends in a curve from the inflow end to the outflow end. The connecting portion 111 is, for example, annular, so that the delivery system can be inserted into the connecting portion 111.

The main section 120 has a cylindrical structure with two open ends, and the cylindrical structure is formed by a plurality of mesh-structured units connected to each other along axial and radial directions. The outer peripheral surface of the main section is configured to abut the inner wall of the left atrium located above the mitral valve annulus, so that blood can flow through the mesh-structured units and reach the inner wall of the left atrium when flowing through the main section. That is to say, the body stent is not covered with a covering, and therefore, when the blood flows through the body stent, a smaller impact force will be applied to the body stent, which is beneficial to increasing the service life of the body stent. Preferably, the mesh-structured unit may be in the form of a diamond, pentagon, hexagon or any other closed shape.

In this embodiment, the main section 120 is, along the axial direction thereof, composed of more than two rows of mesh-structured units that are interconnected in the circumferential direction. The radial diameter of the main section 120 is greater than the maximum radial size of the mitral valve annulus in the diastolic state. In this way, after the artificial heart valve 100 is implanted in the heart, the main section 120 can be attached to the mitral valve annulus so as to prevent the artificial heart valve 100 from shifting. Since there is no covering over the main section, the blood will not be constrained inside the main section, so that the blood will not produce a large impact force on the main section when it flows through the main section, which effectively increases the service life of the main section.

Optionally, a plurality of barbs are provided on the outer peripheral surface of the main section 120, and all the barbs are evenly distributed along the circumferential direction of the main section 120 to assist the main section 120 in anchoring the artificial heart valve to the mitral valve annulus. The inflow end of the barb is connected to the main section 120, and the outflow end of the barb is a free end, and an acute angle is formed between the barb and the main section 120. Alternatively, the outflow end of the barb is connected to the main section 120, and the inflow end of the barb is a free end, and an acute angle is formed between the barb and the main section 120.

The body stent is formed by braiding or cutting nickel-titanium alloy or other biocompatible materials with shape memory properties. It can also be made of elastic or plastically deformable materials, such as balloon-expandable materials.

The artificial valve leaflet 130 includes a valve stent 131, a covering and a clamping component 140. The clamping component 140 is secured to the outer side of the valve stent 131 (i.e., the side of the valve stent 131 away from the axis of the main section), and is configured to clamp the native posterior valve 21. The inflow end of the valve stent 131 is connected to the outflow end of the main section 120, and is overlapped and arranged inside the outflow end of the main section 120; the covering is attached to the outer side of the outflow end of the valve stent 131, so that the outflow end of the valve stent 131 with the covering attached is configured to be in sealing contact with the inner side of the native posterior leaflet 21 of the mitral valve leaflets and mate with the native anterior leaflet 22 of the mitral valve leaflets; and the clamping component 140 is connected to the outer side of the outflow end of the valve stent 131, and the covering exposes the clamping component 140 from the outside so that the native posterior leaflet 21 is clamped and secured between the clamping component 140 and the outflow end of the valve stent 131.

Furthermore, the shape of the surface of the outflow end of the artificial valve leaflet 130 facing the native anterior leaflet (that is, the mating surface of the artificial valve leaflet 130 with the native anterior leaflet) is approximately same as the shape of the surface of the native posterior leaflet facing the native anterior leaflet (that is, the mating surface of the native posterior leaflet with the native anterior leaflet). That is to say, the mating surface of the outflow end of the artificial valve leaflet 130 is shaped according to the mating surface of the native posterior leaflet. Specifically, the valve stent 131 includes a first part 1311 and a second part 1312 that are axially connected, that is, the inflow end of the artificial valve leaflet 130 includes a first part 1311 secured to the outflow end of the body stent; the second part 1312 is attached with the covering, and the outflow end of the artificial valve leaflet 130 includes the second part 1312 and the covering attached thereto, so that the second part 1312 attached with the covering (i.e., the outflow end of the artificial valve leaflet 130) replaces the native posterior leaflet 21 to mate with the native anterior leaflet 22, ensuring the unidirectional flow of blood in the left atrium 10 and left ventricle 20, allowing the artificial valve leaflet 130 to occupy a small area of the valve orifice and bear little impact from blood flow, and effectively prolonging the service life of the artificial heart valve. In detail, the first part 1311 is secured to the inner side of the outflow end of the body stent, the clamping component 140 is provided on the outer side of the second part 1312, and the covering is attached to the outer side of the second part 1312, and the clamping component 140 is exposed outside the covering (that is, exposed on the side of the covering away from the axis of the main section).

After the artificial heart valve 100 is implanted in the heart, the artificial valve leaflet 130 are relatively stationary and are tightly closed or converged by sealing contact with the native posterior leaflet 21. In detail, the second part 1312 extends across the mitral valve annulus into the left ventricle 20 and is in sealing contact with the native posterior leaflet 21. In this way, the second part 1312 replaces the native posterior leaflet 21 to mate with the native anterior leaflet 22, ensuring the unidirectional flow of blood in the left atrium 10 and left ventricle 20. At the same time, the clamping component 140 passes through the chordae tendineae to clamp the native posterior leaflet 21 between the valve stent and the clamping component 140, so as to anchor the second part 1312 attached with the covering to the inner side of the native posterior leaflet 21, thereby preventing the artificial heart valve 100 from shifting under the impact of blood flow.

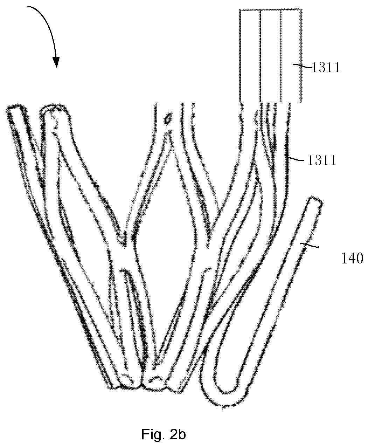

Fig. 2b is a schematic diagram showing the three-dimensional structure of a valve stent according to the present embodiment. As shown in Fig. 2b, the first part 1311 has an arc-shaped structure formed by mesh-structured units connected together, the axial length of the first part 1311 is less than or equal to the axial length of the main section 120, and the radial diameter of the first part 1311 is the same as the radial diameter of the main section. The second part 1312 has an arc-shaped structure formed by a plurality of mesh-structured units connected together. Alternatively, the outflow end of the valve stent has a cylindrical structure with at least one open end, and the cylindrical structure is formed by a plurality of mesh-structured units connected together. Preferably, the mesh-structured units of the first part 1311 and the second part 1312 may be in the form of rhombus, pentagon, hexagon or any other closed shape.

In this case, the radial diameter of the second part 1312 is smaller than the radial diameter of the main section 120 such that the circumferential shape of the second part 1312 matches the circumferential shape of the native posterior leaflet 21. The second part 1312 is connected to the outflow end of the first part 1311 and is located at the middle position of the first part 1311 along the circumferential direction. That is to say, the second part 1312 does not connect both ends of the first part along the circumferential direction.

In an embodiment, the valve stent may be connected to the body stent by means of sutures or welding, that is, the first part 1311 is connected to the body stent by means of sutures or welding. In this embodiment, the mesh-structured units of the first part 1311 are same in shape and size as the mesh-structured units of the outflow end of the body stent, so that the mesh-structured units of the first part 1311 are matched with the respective mesh-structured units of the outflow end of the body stent, so that the mesh-structured units of the first part 1311 can be overlapped with and arranged inside the mesh-structured units of the outflow end of the body stent, and they are connected together at connection points by sutures or welding.

In another embodiment, the first part and the body stent may be integrally formed.

If the second part has an arc-shaped structure, the covering is attached to a side of the outflow end of the valve stent away from the axis of the main section, or the covering covers the outer surface of the outflow end of the valve stent. If the second part has a cylindrical structure, the covering covers the outer surface of the outflow end of the valve stent.

Optionally, if the covering covers the outer surface of the outflow end of the valve stent, the covering is in the shape of a pocket. The covering is filled with filler, and the opening of the covering is closed after the filler is filled. The filler may be biocompatible foam, etc. Optionally, the biocompatible foam includes medical foam silicone rubber, medical polyurethane foam, etc.

The valve stent may be made of metal material or polymer material with relatively high stiffness. The covering may be made of heart valves or pericardial tissue of animals (such as pigs, sheep, horses, and cows), or synthetic materials, such as expanded polytetrafluoroethylene or polyester; the covering may also be made of thermoplastic polycarbonate, polyether urethane, segmented polyether urethane, silicone polyether urethane, silicone-polycarbonate urethane or ultra-high molecular weight polyethylene; the covering may also be made of polyolefins, elastomers, polyethylene glycols, polyethersulfones, polysulfones, polyethylene vinyl pyrrolidone, polyvinyl chloride, fluoropolymers, silicone polyesters, silicone polymers, silicone oligomers, polylactones, or block copolymers using at least two of the above materials.

Optionally, the surface of the covering is treated with or reacted with an anticoagulant which includes but is not limited to heparinized polymers.

The clamping component 140 is secured outside the outflow end of the second part 1312, and the clamping component 140 extends in a circumferential direction away from the axis so as to have a structure shaped substantially as a U shape. For example, it is formed integrally with the second part 1312 by cutting and serves as a part of the second part 1312 extending away from the axis. It may also be connected to the second part 1312 by riveting, welding, etc.

The clamping component 140 is, for example, formed by bending a metal rod. The clamping component 140 has two ends, one of which is connected to the outer side of the second part 1312, and the other of which extends circumferentially away from the axis of the main section and axially away from the inflow end of the valve stent, and then extends circumferentially away from the axis of the main section and axially towards the inflow end of the valve stent, to ensure the area of the native posterior leaflet 21 to be clamped.

The clamping component 140 may be made of nickel-titanium alloy or other biocompatible materials with shape memory properties, or may also be made of elastically or plastically deformable materials, such as balloon-expandable materials, so that the clamping component 140 has shape memory property for clamping and securing the native posterior leaflet 21.

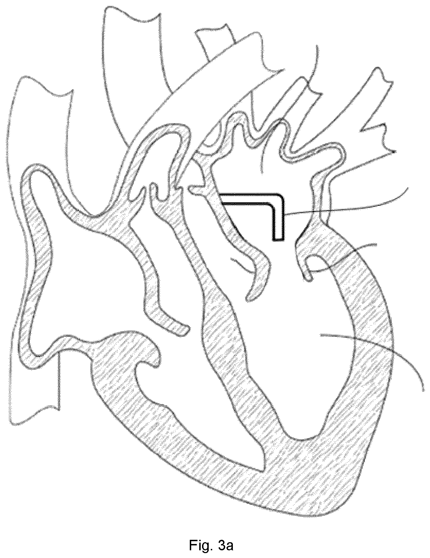

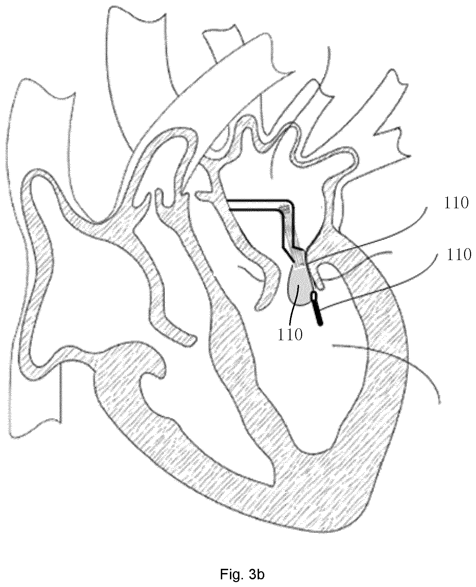

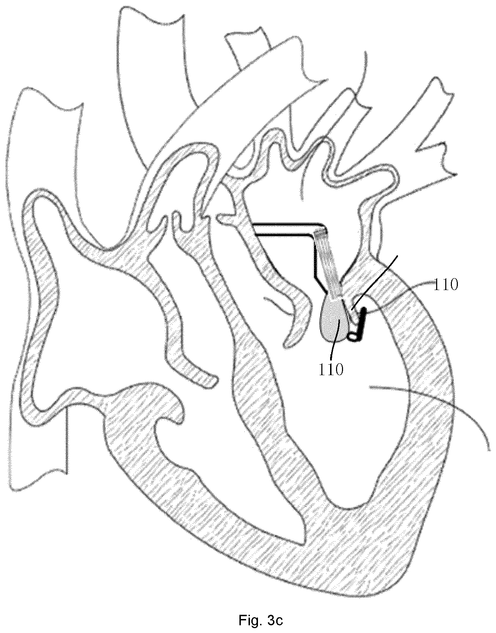

Figs. 3a-3c are schematic diagrams of an artificial heart valve during implantation according to the present embodiment. As shown in Figs. 3a-3c, please also refer to Fig. 1, the implantation process of the artificial heart valve 100 is shown. As shown in Fig. 3a, firstly, the artificial heart valve 100 is loaded on the distal end of the delivery catheter 30 of the delivery system, and the distal end of the delivery catheter 30 is arranged at the inflow end of the lug. The delivery catheter 30 enters the right atrium through the inferior vena cava, and passes through the interatrial septum to the vicinity of the mitral valve annulus of the left atrium 10, so that less trauma incurs in the implantation of the artificial heart valve 100. Then, as shown in Fig. 3b, the delivery catheter 30 begins to release the artificial heart valve 100, and the outflow end of the artificial valve leaflet 130 is released first, and the clamping component 140 located at the second part is released until it passes over the native posterior leaflet 21. At this time, the clamping component 140 does not act and is parallel to the native posterior leaflet 21. As shown in Fig. 3c, as the artificial valve leaflet 130 is released, the clamping component 140 begins to bend and gradually passes through the chordae tendineae and clamps the native posterior leaflet 21 between the clamping component 140 and the artificial valve leaflet 130. At this time, the artificial valve leaflet 130 has occupied the space in which the native posterior leaflet 21 can function, and the native anterior leaflet 22 begins to mate with the relatively stationary artificial valve leaflet 130. As shown in Fig. 1, when the clamping component 140 stably clamps the native posterior leaflet 21 and the artificial valve leaflet 130 is secured in position, the body stent is released and anchored on the mitral valve annulus. The barbs on the body stent and the expanded body stent produce an anchoring force for supporting and securing the body stent to the mitral valve annulus. Then the distal end of the delivery catheter 30 passes through the inflow end of the lug, and is withdrawn from the delivery system to complete the release.

In conclusion, the present invention provides an artificial heart valve, which can be more reliably anchored between the left atrium and the left ventricle through the support of the body stent by the barbs and the mitral valve annulus and the clamping of the native posterior leaflet by the clamping structure. The artificial valve leaflet replaces the native posterior leaflet and mates with the native anterior leaflet, which makes it possible to effectively utilize the healthy native anterior leaflet, thereby improving the mating performance and service life of the artificial valve leaflet. The artificial valve leaflet occupies a small area of the valve orifice, and bears little impact from blood flow, thereby effectively prolonging the service life of the artificial heart valve. In addition, the artificial heart valve can be implanted through the transseptal approach, and therefore less trauma incurs in the implantation.

In addition, it should be noted that unless otherwise specified or pointed out, the terms in the specification "first" and "second" are only used to distinguish various components, elements, steps, etc. in the specification, but is not used to express the logical relationship or sequential relationship between various components, elements, steps, etc.

It can be understood that although the present invention has been disclosed above in preferred embodiments, the above embodiments are not intended to limit the present invention. For any person familiar with the art, without departing from the scope of the technical solution of the present invention, they can use the technical content disclosed above to make many possible changes and modifications to the technical solution of the present invention, or modify it into equivalent examples. Therefore, any simple modifications, equivalent changes and modifications made to the above embodiments based on the technical essence of the present invention without departing from the content of the technical solution of the present invention still fall within the scope of protection of the technical solution of the present invention.

## Claims

1. An artificial heart valve, comprising a body stent and an artificial valve leaflet, wherein an outflow end of the body stent is connected to an inflow end of the artificial valve leaflet, and when the artificial heart valve is implanted in the heart, the body stent is secured to a mitral valve annulus, and the artificial valve leaflet is in sealing contact with an inner side of a native posterior leaflet of a mitral valve leaflet and mates with a native anterior leaflet of the mitral valve leaflet, so as to ensure unidirectional flow of blood in a left atrium and a left ventricle.

2. The artificial heart valve of claim 1, wherein the body stent comprises a lug section and a main section that are connected axially, an inflow end of the main section is connected to an outflow end of the lug section, and an outflow end of the main section is connected to the inflow end of the artificial valve leaflet, and wherein the lug section is configured to connect with a delivery system before the artificial heart valve is released, and the main section is configured to anchor the artificial heart valve to the mitral valve annulus.

3. The artificial heart valve of claim 2, wherein the main section has a cylindrical structure with two open ends, and the cylindrical structure is formed by a plurality of mesh-structured units connected to each other along axial and radial directions, and wherein an outer peripheral surface of the main section is configured to abut against an inner wall of the left atrium located above the mitral valve annulus.

4. The artificial heart valve of claim 2, where a plurality of barbs are provided on an outer peripheral surface of the main section, and all the barbs are evenly distributed along a circumferential direction of the main section to assist the main section in anchoring the artificial heart valve to the mitral valve annulus.

5. The artificial heart valve of claim 2, wherein the lug section is composed of a plurality of lugs, and all the lugs are evenly distributed along a circumferential direction of the main section.

6. The artificial heart valve of claim 5, wherein the lug comprises a connecting portion and two ribs, and wherein the connecting portion is configured to connect to the delivery system, inflow ends of the two ribs are connected to the connecting portion, outflow ends of the two ribs are connected to the main section, and the ribs each extend in a curve from the inflow end to the outflow end.

7. The artificial heart valve of claim 1, wherein the artificial valve leaflet comprises a valve stent, a covering and a clamping component;
an inflow end of the valve stent is connected to the outflow end of the main section, and is overlapped and arranged inside the outflow end of the main section;
the covering is attached to an outer side of an outflow end of the valve stent, so that an outflow end of the valve stent with the covering attached is configured to be in sealing contact with an inner side of the native posterior leaflet of the mitral valve leaflet and mate with the native anterior leaflet of the mitral valve leaflet; and
the clamping component is connected to the outer side of the outflow end of the valve stent, and the covering exposes the clamping component from outside so that the native posterior leaflet is clamped and secured between the clamping component and the outflow end of the valve stent.

8. The artificial heart valve of claim 7, wherein the inflow end of the valve stent has an arc-shaped structure formed by a plurality of mesh-structured units connected together, and a radial diameter of the inflow end of the valve stent is same as a radial diameter of the main section.

9. The artificial heart valve of claim 7, wherein the outflow end of the valve stent has an arc-shaped structure formed by a plurality of mesh-structured units connected together; or the outflow end of the valve stent has a cylindrical structure with at least one open end, and the cylindrical structure is formed by a plurality of mesh-structured units connected together;
wherein a radial diameter of the outflow end of the valve stent is smaller than a radial diameter of the inflow end of the valve stent.

10. The artificial heart valve of claim 9, wherein the mesh-structured units of the inflow end of the valve stent are matched with and arranged inside the mesh-structured units of the outflow end of the body stent, and the mesh-structured units of the inflow end of the valve stent are connected to the mesh-structured units of the outflow end of the body stent at overlapped connecting points.

11. The artificial heart valve of claim 10, wherein the mesh-structured units of the inflow end of the valve stent are same in shape and size as the mesh-structured units of the outflow end of the body stent.

12. The artificial heart valve of claim 9, wherein
the outflow end of the valve stent has the arc-shaped structure, the covering is attached to a side of the outflow end of the valve stent away from an axis of the main section, or the covering covers an outer surface of the outflow end of the valve stent; or
the outflow end of the valve stent has the cylindrical structure, the covering covers an outer surface of the outflow end of the valve stent.

13. The artificial heart valve of claim 12, wherein the covering covers an outer surface of the outflow end of the valve stent, and the covering is filled with filler.

14. The artificial heart valve of claim 13, wherein the filler comprises biocompatible foam.

15. The artificial heart valve of claim 7, wherein the clamping component is formed by bending a metal rod, the clamping component has two ends, one of which is connected to the outer side of the outflow end of the valve stent, and the other of which extends circumferentially away from an axis of the main section and axially away from the inflow end of the valve stent, and then extends circumferentially away from the axis of the main section and axially towards the inflow end of the valve stent.
